# EUROPEAN PATENT SPECIFICATION

(11) **EP 2 414 541 B1**
(45) Date of publication and mention of the grant of the patent: **07.05.2014**
(21) Application number: 10712993.4
(22) Date of filing: 25.03.2010
(51) Int. Cl.: C12Q 1/68

(54) **METHYLATION LIGATION-DEPENDENT MACROARRAY (MLM)**
METHYLIERUNGS-/LIGATIONSABHÄNGIGER MAKROARRAY (MLM)
MACROPUCE POUR L'IDENTIFICATION DE MÉTHYLATIONS DÉPENDANTE D'UNE LIGATURE (MLM)

(30) Priority: 31.03.2009 US 202732 P
(43) Date of publication of application: 08.02.2012
(73) Proprietor: Centre Hospitaller Universitaire Vaudois (CHUV), 1011 Lausanne (CH)
(72) Inventor: BENHATTAR, Jean, CH-1009 Pully (CH); GUILLERET, Isabelle, CH-1110 Morges (CH)
(74) Representative: KATZAROV S.A.
(86) International application number: PCT/IB2010/051313
(87) International publication number: WO 2010/113088

(56) References cited:
- EP-A1- 1 130 113
- WO-A1-2007/025594
- WO-A2-01/92579
- US-A1- 2007 092 883
- NYGREN ANDERS O H ET AL: "Methylation-specific MLPA (MS-MLPA): simultaneous detection of CpG methylation and copy number changes of up to 40 sequences" NUCLEIC ACIDS RESEARCH, vol. 33, no. 14, 2005, XP002596755 ISSN: 0305-1048
- JEUKEN JUDITH WM ET AL: "MS-MLPA: an attractive alternative laboratory assay for robust, reliable, and semiquantitative detection of MGMT promoter hypermethylation in gliomas" LABORATORY INVESTIGATION, vol. 87, no. 10, October 2007 (2007-10), pages 1055-1065, XP002596756 ISSN: 0023-6837
- SCHOUTEN J P ET AL: "Relative quantification of 40 nucleic acid sequences by multiplex ligation-dependent probe amplification" NUCLEIC ACIDS RESEARCH, OXFORD UNIVERSITY PRESS, SURREY, GB LNKD- DOI:10.1093/NAR/GNF056, vol. 30, no. 12, 15 June 2002 (2002-06-15) , pages 1-13, XP002362868 ISSN: 0305-1048

## Description

### FIELD OF THE INVENTION

The invention relates to the filed of biochemistry, namely to testing process involving nucleic acids. In particular, the invention relates to a method for detecting the presence of a methylated site at a specific location on a single stranded target nucleic acid sequence, to nucleic acid probes for use in the method.

### BACKGROUND OF THE INVENTION

Alterations of DNA methylation patterns have been recognized as a common change in human cancers. Aberrant methylation of normally unmethylated CpG-rich areas, also known as CpG-islands, which are located in or near the promoter region of many genes, have been associated with transcriptional inactivation of important tumor suppressor genes, DNA repair genes, and metastasis inhibitor genes. Therefore, detection of aberrant promoter methylation of cancer-related genes may be essential for diagnosis, prognosis and/or detection of metastatic potential of tumors. As the number of genes known to be hypermethylated in cancer is large and increasing, sensitive and robust multiplex methods for the detection of aberrant methylation of promoter regions are therefore desirable. In addition, the amount of DNA available for large-scale studies is often limited and can be of poor quality, for example DNA isolated from formalin treated, paraffin-embedded tissues that have been stored at room temperature for years.

One method for detection of target nucleic acid sequences is disclosed in WO 96/15271 (ABBOTT LABORATORIES). This method relates to amplifying and detecting a target nucleic acid sequence and, more particularly, to a method for specifically amplifying multiple target nucleic acid sequences using a single pair of primers. However this method is not suitable for the simultaneous analysis of methylation changes in a large number of genes.

The Multiplex Ligation-dependent Probe Amplification (MLPA) technique (WO 01/61033, SCHOUTEN JOHANNES PETRUS) is a method for multiplex detection of copy number changes of genomic DNA sequences using DNA samples derived from blood. This method detects the presence and quantifies at least one specific single stranded target nucleic acid sequence in a sample using a plurality of probe sets of at least two probes, each of which includes a target specific region and a non-complementary region comprising a primer binding site. The probes belonging to the same set are ligated together when hybridised to the target nucleic acid sequence and amplified by a suitable primer set.

A method for multiplex detection and quantification of target nucleic acid sequences and variations thereof in a sample is disclosed in WO 2007/025594 A1 (PAMGENE B.V.). This method relates to an assay for detection of SNPs (single nucleotide polymorphisms) and nucleic acid copy numbers, by using solid supports / arrays.

The Methylation-Specific Multiplex Ligation-dependent Probe Amplification (MS-MLPA) (US 2007/0092883, DE LUWE HOEK OCTROOIEN B.V.; Nygren Andres et al., "Methylation-specific MLPA (MS-MLPA): simultaneous detection of CpG methylation and copy number changes of up to 40 sequences", Nucleic Acids Research, 2005, 33(14)) can detect changes in both CpG methylation as well as copy number of up to 40 chromosomal sequences in a simple reaction. MS-MLPA method detects the presence of specific methylated sites in a single stranded target nucleic acid, while simultaneously, the quantification of the target nucleic acid sequence can be performed, using a plurality of probe sets of at least two probes, each of which includes a target specific region and non-complementary region containing a primer binding site. At least one of the probes further includes the sequence of one of the strands of a double stranded recognition site of a methylation sensitive restriction enzyme. The probes belonging to the same set are ligated together when hybridised to the target nucleic acid sequence, the hybrid is subjected to digestion by the methylation sensitive restriction enzyme, resulting in non-methylated recognition sites being cleaved. The probes of the uncleaved (methylated) hybrid are subsequently amplified by PCR with a suitable primer set. In MS-MLPA, the various amplicons, obtained after the PCR amplification, are discriminated on the basis of size after size fractionation on a gel. However, the discrimination of amplicons which differ only slightly in size is difficult and could lead to artefactual signal. Moreover, the PCR amplification of longer DNA fragments is slower in respect to shorter DNA fragments. Thus the PCR amplification is not homogenous. Another difficulty with the amplicons discrimination on the basis of size is that it is very hard to add new MLPA probes having new and different nucleic acid sequences.

Therefore there is still a need to develop a simple method for the simultaneous analysis of methylation changes in a large number of different genomic DNA sequences. This method should be also easily adapted to any target nucleic acid sequence.

### SUMMARY OF THE INVENTION

This object has been achieved by the Applicants in the present invention which provides a method for detecting in a sample comprising at least one nucleic acid, the presence of at least one methylated site on a specific location in a specific single stranded target nucleic acid sequence comprising a first and a second segment, said segments located adjacent to one another, and said method comprising, in a reaction mixture, the steps of:
1) incubating said sample, comprising at least one nucleic acid, with a plurality of different probe sets, under conditions allowing hybridization between complementary target nucleic acid sequences in said nucleic acid with said probe sets, each probe set comprising
   a first nucleic acid probe having
      a first target specific region complementary to the first segment of said target nucleic acid sequence and
      a first non-complementary region, 3' from the first region, being non-complementary to said target nucleic acid sequence, and comprising a tag sequence,
   a second nucleic acid probe having
      a second target specific region complementary to the second segment of said target nucleic acid sequence, phosphorylated at the 5' extremity, and
      a second non-complementary region, 5' from the second region, being non-complementary to said target nucleic acid sequence, and comprising a tag sequence,
   and obtaining said first and said second nucleic acid probe of each probe set being hybridized to the complementary first and second segment of the same target nucleic acid sequence, respectively said hybridized first and second nucleic acid probes of each probe set being located adjacent to one another,
2) connecting to one another the first and the second nucleic acid probes hybridized to target nucleic acid sequences, which provides a plurality of different connected probe assemblies of substantially the same size, and forming a double-stranded connected probe assemblies - target sequence, wherein the sequence of at least one of the nucleic acid probes is chosen such that, in the connected probe assembly, at least one double-stranded recognition site for a methylation sensitive restriction enzyme is present,
3) incubating said connected probe assemblies - target sequence with at least one methylation sensitive restriction enzyme, allowing said methylation sensitive restriction enzyme to cleave said double-stranded connected probe assemblies - target sequence at unmethylated recognition sites, leaving methylated recognition sites intact,
4) amplifying the connected probe assemblies, wherein amplification is initiated by binding of a first amplification primer specific for a tag sequence of said first nucleic acid probe, followed by elongation, whereby producing amplicons of substantially the same size
5) immobilizing capture probes, having a sequence complementary to at least about half of the sequences of both the first and the second nucleic acid probes, to a support,
6) contacting simultaneously all amplicons of step 4 with said capture probes under conditions effective to hybridize said amplicons to said capture probes.
7) detecting said amplicons, wherein said detecting indicates the presence in the sample of one or more methylated sites on specific location in specific single stranded target nucleic acid sequence.

### BRIEF DESCRIPTION OF THE FIGURES

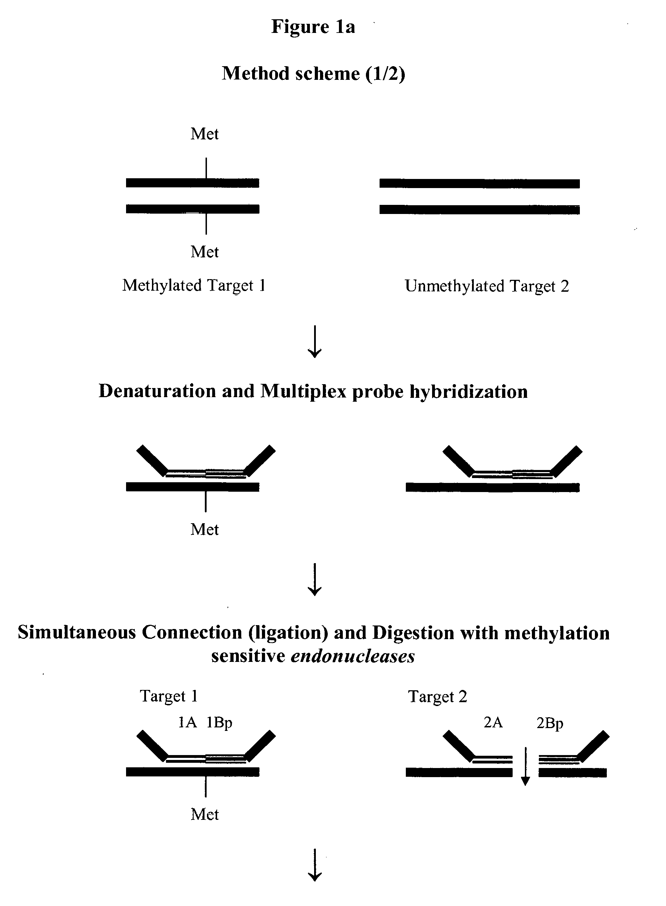

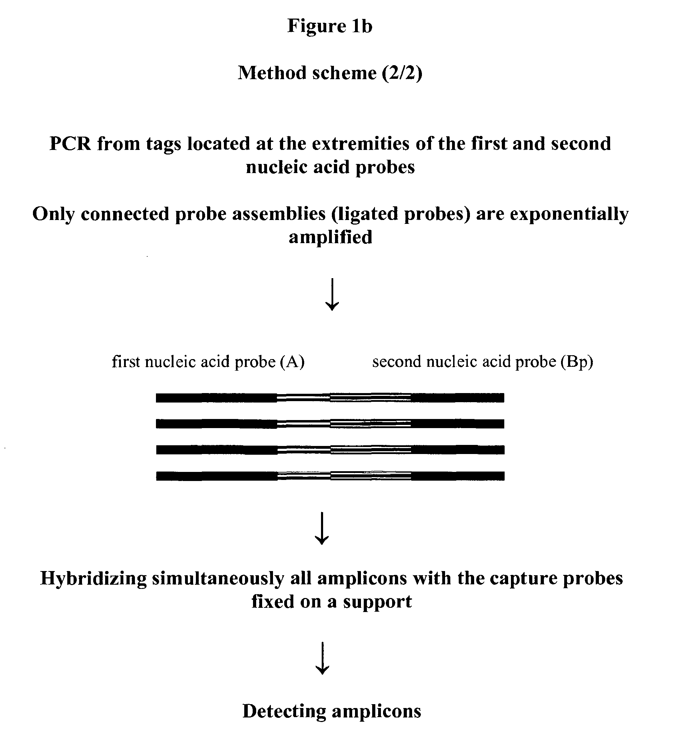

**Figure 1a** **and** **1b** present Methylation Ligation-dependent Macroarray (MLM) method scheme

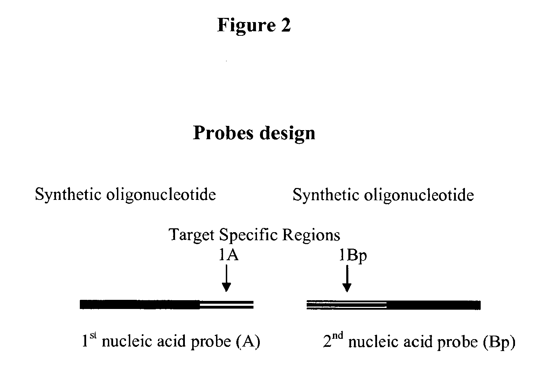

**Figure 2** shows the design of the nucleic acid probe of the invention

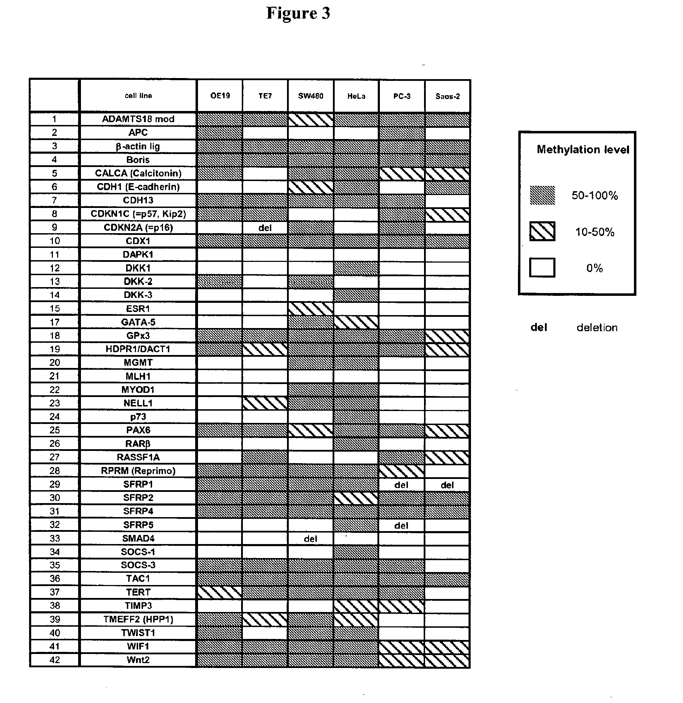

**Figure 3** illustrates experimental result showing the methylation status of 42 promoter genes in 6 human cancer cell lines (OE19 and TE7: esophageal cancers, SW480: colon cancer; HeLA: cervical cancer; PC-3: prostate cancer; Saos-2: osteosarcoma)

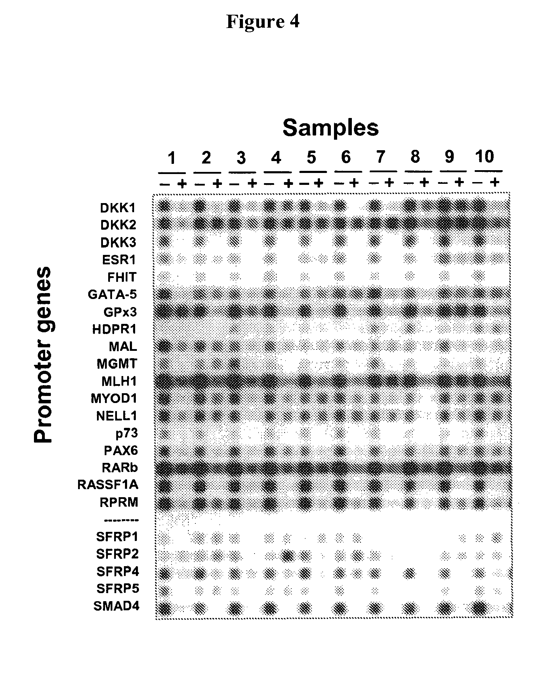

**Figure 4** presents experimental result showing a portion of a macroarray with the methylation analysis of 23 promoter genes in 10 colorectal tumor samples. After digestion with the CfoI methylation-sensitive enzyme, Taq ligase joined primers annealed to the methylated sequence. Ligation products are then amplified and tagged in a PCR reaction. These PCR products are finally hybridized to the macroarray (lane +). For each sample, a control without digestion (lane -) is included to check the correct amplification of all targets, which led to a full positive signal on the macroarray.
**Figure 5** shows immobilizing of capture probes on a membrane

### DETAILED DESCRIPTION OF THE INVENTION

In the case of conflict, the present specification, including definitions, will control.
Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise" is generally used in the sense of include, that is to say permitting the presence of one or more features or components.

As used in the specification and claims, the singular form "a", "an" and "the" include plural references unless the context clearly dictates otherwise.

As used herein, the term "DNA polymorphism" refers to the condition in which two or more different nucleotide sequences can exist at a particular site in the DNA. "SNP" stands for single nucleotide polymorphism.

As used herein, "oligonucleotide" indicates any short segment or sequence of nucleic acid having a length between 10 up to at least 800 nucleotides. Oligonucleotides can be generated in any matter, including chemical synthesis, restriction endonuclease digestion of plasmids or phage DNA, DNA replication, reverse transcription, or a combination thereof. One or more of the nucleotides can be modified e.g. by addition of a methyl group, a biotin or digoxigenin moiety, a fluorescent tag or by using radioactive nucleotides.

As used herein, the term "primer" refers to an oligonucleotide, whether occurring naturally as in a purified restriction digest or produced synthetically, which is capable of acting as a point of initiation of nucleic acid sequence synthesis when placed under conditions in which synthesis of a primer extension product which is complementary to a nucleic acid strand is induced, i.e. in the presence of different nucleotide triphosphates and a polymerase in an appropriate buffer ("buffer" includes pH, ionic strength, cofactors etc.) and at a suitable temperature. One or more of the nucleotides of the primer can be modified for instance by addition of a methyl group, a biotin or digoxigenin moiety, a fluorescent tag or by using radioactive nucleotides. A primer sequence need not reflect the exact sequence of the template. For example, a non-complementary nucleotide fragment may be attached to the 5' end of the primer, with the remainder of the primer sequence being substantially complementary to the strand.

As used herein, the terms "target sequence" or "target nucleic acid sequence" refer to a specific nucleic acid sequence wherein methylation changes are to be detected. Target sequence or target nucleic acid sequence can be derived for example from genomic DNA.

As used herein, "amplification" refers to the increase in the number of copies of a particular nucleic acid sequence. Copies of a particular nucleic acid sequence made in vitro in an amplification reaction are called "amplicons" or "amplification products".

As used herein, "probe" refers to a known sequence of a nucleic acid that is capable of selectively binding to a target nucleic acid. Additionally a "ligated probe" or "connected probe assemblies" refers to the end product of a ligation reaction between a pair of probes.

As used herein, the term substantially "adjacent" is used in reference to nucleic acid molecules that are in close proximity to one another. The term also refers to a sufficient proximity between two nucleic acid molecules to allow the 5' end of one nucleic acid that is brought into juxtaposition with the 3' end of a second nucleic acid so that they may be connected (ligated) by a ligase enzyme. Nucleic acid segments are defmed to be substantially adjacent when the 3' end and the 5' end of two probes, one hybridising to one segment and the other probe to the other segment, are sufficiently near each other to allow connection of the ends of both probes to one another. Thus, two probes are substantially adjacent, when the ends thereof are sufficiently near each other to allow connection of the ends of both probes to one another.

As used herein, the terms "detected", "detecting" and "detection" are used interchangeably and refer to the discernment of the presence or absence of an amplicon, wherein detecting of an amplicon indicates the presence in the sample of one or more methylated sites on specific location in specific single stranded target nucleic acid sequence.

As used herein, the terms "restriction endonucleases" and "restriction enzymes" refer to enzymes, such as bacterial enzymes or recombinant enzymes, each of which cut double-stranded DNA at or near a specific nucleotide sequence, which sequence is referred to as "recognition site", or "double-stranded recognition site".

As used herein the term "PCR" refers to the polymerase chain reaction (Mulis et al U.S. Pat. Nos. 4,683,195, 4,683,202 and 4,800,159). The PCR amplification process results in the exponential increase of discrete DNA fragments whose length is defined by the 5' ends of the oligonucleotide primers.

As used herein, "sample" comprises at least one nucleic acid, preferably a plurality of different nucleic acids. These nucleic acids are derived for example from genomic DNA of different subjects or of the same one subject.

As used herein, the terms "hybridisation" and "annealing" are used in reference to the pairing of complementary nucleic acids.

As used herein, the term "complementary" means that two nucleic acids, e.g. DNA or RNA, contain a series of consecutive nucleotides which are capable of forming base pairs to produce double-stranded region referred to as a hybridization duplex or complex. A duplex forms between nucleic acids because of the orientation of the nucleotides on the RNA or DNA strands; certain bases attract and bond to each other to form a base pair through hydrogen bonding and π-stacking interactions. Thus, adenine in one strand of DNA or RNA pairs with thymine in an opposing complementary DNA strand, or with uracil in an opposing complementary RNA strand. Guanine in one strand of DNA or RNA pairs with cytosine in an opposing complementary strand.

As used herein the terms "subject" or "patient" are well-recognized in the art, and, are used interchangeably herein to refer to a mammal, including dog, cat, rat, mouse, monkey, cow, horse, goat, sheep, pig, camel, and, most preferably, a human. In some embodiments, the subject is a subject in need of treatment or a subject with a disease or disorder. However, in other embodiments, the subject can be a normal subject. The term does not denote a particular age or sex. Thus, adult and newborn subjects, as well as fetuses, whether male or female, are intended to be covered.

In one embodiment, the present invention provides for a method for detecting in a sample comprising at least one nucleic acid, the presence of at least one methylated site on a specific location in a specific single stranded target nucleic acid sequence comprising a first and a second segment, said segments located adjacent to one another, and said method comprising, in a reaction mixture, the steps of:
1) incubating said sample, comprising at least one nucleic acid, with a plurality of different probe sets, under conditions allowing hybridization between complementary target nucleic acid sequences in said nucleic acid with said probe sets, each probe set comprising
   a first nucleic acid probe having
      a first target specific region complementary to the first segment of said target nucleic acid sequence and
      a first non-complementary region, 3' from the first region, being non-complementary to said target nucleic acid sequence, and comprising a tag sequence,
   a second nucleic acid probe having
      a second target specific region complementary to the second segment of said target nucleic acid sequence, phosphorylated at the 5' extremity, and
      a second non-complementary region, 5' from the second region, being non-complementary to said target nucleic acid sequence, and comprising a tag sequence,
   and obtaining said first and said second nucleic acid probe of each probe set being hybridized to the complementary first and second segment of the same target nucleic acid sequence, respectively said hybridized first and second nucleic acid probes of each probe set being located adjacent to one another,
2) connecting to one another the first and the second nucleic acid probes hybridized to target nucleic acid sequences, which provides a plurality of different connected probe assemblies of substantially the same size, and forming a double-stranded connected probe assemblies - target sequence, wherein the sequence of at least one of the nucleic acid probes is chosen such that, in the connected probe assembly, at least one double-stranded recognition site for a methylation sensitive restriction enzyme is present,
3) incubating said connected probe assemblies - target sequence with at least one methylation sensitive restriction enzyme, allowing said methylation sensitive restriction enzyme to cleave said double-stranded connected probe assemblies - target sequence at unmethylated recognition sites, leaving methylated recognition sites intact,
4) amplifying the connected probe assemblies, wherein amplification is initiated by binding of a first amplification primer specific for a tag sequence of said first nucleic acid probe, followed by elongation, whereby producing amplicons of substantially the same size
5) immobilizing capture probes, having a sequence complementary to at least about half of the sequences of both the first and the second nucleic acid probes, to a support,
6) contacting simultaneously all amplicons of step 4 with said capture probes under conditions effective to hybridize said amplicons to said capture probes.
7) detecting said amplicons, wherein said detecting indicates the presence in the sample of one or more methylated sites on specific location in specific single stranded target nucleic acid sequence.

Preferably, the sample of step 1) comprises a plurality of different nucleic acids.

In the method of the present invention, said different nucleic acids have homogenized sizes and are obtained usually by sonication of the DNA, preferably genomic DNA, in an ultrasounds bath during approximately 30 minutes. Other methods for the DNA, preferably genomic DNA, denaturation and fragmentation known to those skilled in the art can be also used in the method of the present invention.

A complementary nucleic acid is capable of hybridising to another nucleic acid under normal hybridisation conditions. It may comprise mismatches at a small minority of the sites. Hybridization traditionally is understood as the process by which two partially or completely complementary strands of nucleic acid are allowed to come together in an antiparallel fashion (one oriented 5' to 3', the other 3' to 5') to form a double-stranded nucleic acid with specific and stable hydrogen bonds, following explicit rules pertaining to which nucleic acid bases may pair with one another. The hybridization mixture used in hybridization can be different in its composition than what has been presented later in the working Examples, for example, the salt composition and/or the concentration can vary, or commercially available hybridization solutions can be used (such as ArrayHyb, Sigma). In addition, denaturing or stabilizing additives (such as formamide, DMSO, i. e. dimethyl sulfoxide) or substances that decrease non-specific binding (such as BSA, i. e. bovine serum albumin, or ssDNA, i. e. salmon sperm DNA) can be used in the hybridization mixture. Hybridization can be carried out in various hybridization temperatures (generally between 40-70°C), and the time needed to perform hybridization can vary, depending on the application, from a few minutes to one day. Instead of a water bath, hybridization can be carried out, e. g. , in an incubator or in a special hybridization device (e. g. , GeneTAC HybStation or Lucidea Slidepro Hybridizer).
For example normal hybridization conditions for nucleic acid of approximately 10 to 250 nucleotides would usually include a temperature of approximately 60°C in the presence of 1.08M sodium chloride, 60 mM sodium phosphate and 6 mM ethylenediamine tetraacetic acid (pH 7.4). Hybridization conditions are easily modified to suit nucleic acids of differing sequences. Reaction parameters which are commonly adjusted are the concentration and type of ionic species present in the hybridization solution, the types and concentrations of denaturing agents present, and the temperature of hybridization. Factors which may influence the hybridization conditions for a particular nucleic acid composition are base composition of the probe/target duplex, as well as by the level and geometry of mispairing between the two nucleic acids.

In the method of the present invention, at least steps 2 and 3 are performed simultaneously or sequentially. Preferably the connecting (ligation) step 2 of the nucleic acid probes when hybridized to their target nucleic acid sequence is combined with simultaneous digestion (step 3) of these double-stranded connected probe assemblies - target sequence (double-stranded MLM probe assemblies/DNA) with methylation-sensitive restriction endonucleases such as, but not limited to HhaI (Cfo I) or HpaII. The methods for connecting (ligation) and digestion are those known to the person skilled in the art.

In case the ligation and the digestion with the methylation sensitive restriction enzyme are performed simultaneously, which is preferred, the temperature in such a combined ligation/digestion step should be chosen such that both the ligation activity and the methylation sensitive restriction endonuclease activity are efficient. For example, in case HhaI is used as methylation sensitive restriction endonuclease, the temperature is usually lower or equal to 54°C, preferably the temperature is 49°C and the most preferably 37°C. It was found that HhaI activity decreases at temperatures above 50°C. Further, to ensure complete digestion of the double-stranded MLM probe assemblies/DNA, the ligation and digestion time should be adjusted accordingly, which is easily performed without any inventive skill by the skilled person.

The target sequences detected by MLM probes of the present invention contain a restriction site recognized by a methylation sensitive endonuclease, such as, but not limited to HhaI or HpaII, that are sensitive to cytosine methylation of one CpG site in their recognition sequence. Upon digestion with one of these enzymes, a probe amplification product will only be obtained if the CpG site is methylated.

Furthermore, a plurality of different methylation sensitive restriction enzymes can be used in a single or in separate reactions to detect multiple methylated sites within a single or a plurality of target nucleic acid sequences.

Digestion of the double-stranded MLM probe assemblies/DNA, rather than double stranded genomic DNA, allowed the use of DNA derived from formalin treated paraffin-embedded tissue samples, thus enabling retrospective studies.

It is to be understood that any methylated site present in a target nucleotide sequence can be detected by MLM probes according to the present invention, as long as the site of interest (as part of a double stranded recognition sequence) can be recognized by a methylation sensitive restriction endonuclease, cleaving the nucleic acid when the sequence of interest is unmethylated, and leaving the nucleic acid uncleaved when the sequence is methylated.

The complete digestion is apparent by the disappearance of at least some MLM probes in a MLM reaction whereas incomplete digestion results in general background peak signals of all MLM probes.

In another aspect, the invention provides the use of a nucleic acid probe (MLM probe) in the method of the present invention, comprising a single stranded nucleic acid sequence, constituting one of the strands of the double stranded recognition site of the methylation sensitive restriction enzyme. Such a nucleic acid probe can be used according to the invention, by hybridizing to a complementary sequence in a target nucleic acid sequence, and creating a double-stranded recognition site for the methylation sensitive restriction enzyme, enabling the above-mentioned restriction enzyme to cleave, if the target nucleic acid is not methylated.

In a further embodiment of the present invention, the nucleic acid probe (MLM probe) comprises at least at one of the termini thereof, at least a part of a single stranded sequence, constituting one of the strands of a double stranded recognition site of the methylation sensitive restriction enzyme. Such a probe can still provide for one of the strands of a double-stranded recognition site for the methylation sensitive restriction enzyme, when the said terminus is connected, by the method according to the invention, with the terminus of another probe, providing the missing portion of the said recognition sequence. By connecting (ligation) of the said probes, the required recognition sequence is created, and the double stranded probe assembly, formed with the said probes in the claimed method, provides the double-stranded recognition site for the methylation sensitive restriction enzyme. One of the strands of a double stranded recognition site of the methylation sensitive restriction enzyme is formed when the 3' end of the first nucleic acid probe is connected to the 5' end of the second nucleic acid probe.

In the method of the present invention, DNA, for example genomic DNA, is first fully denatured, followed by the formation of a hemimethylated DNA complex with the MLM probes of the invention. Methylation of only the simple DNA strand of this complex is sufficient to inhibit methylation-sensitive digestion. This is in line with earlier reports, which demonstrated that methylation of one strand is sufficient to block digestion by most methylation-sensitive restriction endonucleases (Bird, A. P. (1978) Use of restriction enzymes to study eukaryotic DNA methylation: II. The symmetry of methylated sites supports semi-conservative copying of the methylation pattern. J. Mol.Biol., 118, 49-60; Gruenbaum, Y., Cedar, H. and Razin, A. (1981) Restriction enzyme digestion of hemimethylated DNA. Nucleic Acids Res., 9, 2509-2515).

When designing the MLM probes of the invention, for example for detection of methylation in CpG islands, it is highly preferred that one methylation-sensitive restriction site is present within the target (recognition) sequence, because not all CpG sites in a CpG island need to be methylated to silence the transcription of a particular gene (Tischkowitz, M., Ameziane, N., Waisfisz, Q., De Winter, J. P., Harris, R., Taniguchi, T., D'Andrea, A., Hodgson, S. V., Mathew, C. G. and Joenje, H. (2003) Bi-allelic silencing of the Fanconi anaemia gene FANCF in acute myeloid leukaemia. Br.J.Haematol., 123, 469-471; Taniguchi, T., Tischkowitz, M., Ameziane, N., Hodgson, S. V., Mathew, C. G., Joenje, H., Mok, S. C. and D'Andrea, A. D. (2003) Disruption of the Fanconi anemia-BRCA pathway in cisplatin-sensitive ovarian tumors. Nat.Med., 9, 568-574). Thus, if a signal is generated from one MLM probe but not from a second probe located elsewhere in the same promoter, this indicates that the particular gene is methylated and additional tests should be performed.

The amount of at least the first nucleic acid probe of each probe set in the annealing mixture is less than 4 femtomole, preferably 0.75 femtomole. The probe sets differ from one another in that at least one of the probes of different probe sets have different target specific regions, therewith implicating that each probe set is specific for a unique target nucleic acid sequence. However, probe sets may also only differ in one of the probes, the other probe(s) being identical. Such primer sets can be used for example for the determination of a specific point mutation or polymorphism or for the determination of splice variants in the sample nucleic acids.

Preferably, the molar amount of at least the first nucleic acid probe of at least one probe set, preferably of a plurality of probe sets, more preferably of each probe set in the mixture is less than 1 femtomole. By such low probe amounts, reliable amplification of up to for example 50 different sets of probes can be achieved.

Preferably, the nucleic acid probes of the same probe set are present in the mixture in substantially equal amounts, although the said amounts can differ from one another, for example depending on the hybridisation characteristics of the target specific regions with the target nucleic acid sequence. However, the amount of second nucleic acid probe may optionally be a factor 1-5 higher than that of the corresponding first nucleic acid probe, without negatively affecting the reaction.

Although it is possible for the first nucleic acid probe of different probe sets to have different tag sequences, implicating that a plurality of different first amplification primers are to be used in the amplification step it is highly preferred that the first tag sequences of the first nucleic acid probes of the different probe sets are identical, so that only one first amplification primer has to be used in the amplification reaction. A bias in the amplification due to a difference in the sequence of different primers used for the amplification can thus be completely avoided, resulting in a substantially uniform amplification for all probe assemblies. According to the invention it is however also possible that a number of first nucleic acid probes comprise the same tag sequence, whereas first probes belonging to another probe set may comprise another first tag sequence.

According to another embodiment of the present invention, the amplification (step 4) comprises binding of a second amplification primer, specific to a tag sequence of said second nucleic acid probe, to the elongation product of the first primer. By the use of a second amplification primer, the amplification reaction is not linear, but exponential. Said first and said second probe preferably each comprise a different tag. Preferably said amplification of connected probes is performed with the use of the Polymerase Chain Reaction (PCR). Alternatively other amplification methods for nucleic acids such as the 3SR (Self-sustained sequence replication) (Genome Res. 1991. 1: 25-33) and NASBA (Nucleic acid sequence-based amplification) (EP0629706B1) techniques are also compatible with the current invention.

In line with the above, preferably the second tag sequences of the second nucleic acid probes of the different probe sets are identical, so that for amplification of the primer assemblies a limited amount of different primers may be used. In this way, amplification of all possible primer assemblies can be accomplished using a limited number of amplification primer pairs, preferably only one amplification primer pair. As in such a case, all the probes comprise the same first tag and the same second tag, thereby excluding any bias in the amplification of the probes due to sequence differences in the amplification primers.

According to the present invention, said first nucleic acid amplification primer and/or said second nucleic acid amplification primer is tagged with a labelled tag. Thus one or more of the amplification primers can be modified and tagged for example by addition of a methyl group, a biotin or digoxigenin moiety, a fluorescent tag or by using radioactive nucleotides. Preferably the labelled tag is selected in the group comprising a fluorescent tag, non-fluorescent tag (such as biotin), radioactive tag, luminescent tag and phosphorescent tag. More preferably at least one nucleic acid amplification primer is tagged at its 5' end with biotine.

However, it is of course possible to use probes that comprise different first tags and/or different second tags. In this case it is preferred that the amplification primers are matches for similar priming efficiencies. However, some bias can be tolerated for non quantitative applications or when the bias is known, it can be taken into account in a quantitative application.

Because of the preferred low amounts of probes present in the reaction mixture, the number of different probe sets in one reaction may exceed the maximum number of probe sets that can be achieved with the multiplex methods known in the art. The reaction mixture comprises at least one probe set, preferably at least 2 probe sets, more preferably at least 10 probe sets, even more preferably at least 20 probe sets, the most preferably 40 probe sets and even most preferably up to 50 different probe sets. It is to be understood that it is preferred to use lower probe amounts when the number of different probe sets increases. Using for example 10 different probe sets, the amount of each first probe is preferably less than 5-20 femtomoles, whereas when 30-50 different probe sets are used, the amount of each different first probe is preferably in the range of 0.5-2 femtomoles in the reaction mixture.

The presence of a second, or further additional, distinct methylated target nucleic acid can be detected with the method according to the present invention. To enable this it is possible that said sample is provided with at least two probe sets, i.e. the target specific regions of at least one of the first, second, or, when present, the third nucleic acid probes of each set differ from one another. In this case at least two different amplicons can be detected. For example when a first or said second nucleic acid probe of a probe set is capable of hybridising to (methylated) target nucleic acid essentially adjacent to a probe of the second probe set. Successful connecting of probes can then result in an amplicon resulting from the connection of said first and said second nucleic acid probe of the first set and an amplicon resulting from the connection of the first and second of the second set. For enabling detection of each additional target nucleic acid one can similarly provide additional probes. This has applications for the detection and relative quantification of more than one target nucleic acid which need not be in the same chromosomal region.

To allow connection of essentially adjacent probes through ligation, the probes preferably do not leave a gap upon hybridisation with the target sequence. In that case the first and second segments of the target nucleic acids are adjacent. However, it is also possible that between the first and second segments a third segment is located on the target nucleic acid. In that case a third probe may be provided in a probe set complementary to the third segment of said target nucleic acid, whereby hybridisation of the third probe to said third segment allows the connecting of the first, second and third probes. In this embodiment of the invention a gap upon hybridisation of the first and second probes to the target nucleic acid is filled through the hybridisation of the third probe. Upon connecting and amplification, the resulting amplicon will comprise the sequence of the third probe. One may choose to have said interadjacent part to be relatively small thus creating an increased difference in the hybridisation efficiency between said third segment of the target nucleic acid and the third nucleic acid probe that comprises homology with said third segment of said target nucleic acid, but comprises a sequence which diverges from the perfect match in one or more nucleotides. In another embodiment of the invention a gap between first and second probes on said target nucleic acid is filled through extending a 3' end of a hybridised probe or an additional nucleic acid filling part of an interadjacent part, prior to said connecting.

The nucleic acid probes, not hybridised in the incubation step are not removed in the course of the method according to the invention and remain in the reaction mixture together with the hybridised nucleic acid probes. In the method of the present invention, reaction conditions are used that do not require unligated probe removal or buffer exchange.

It is preferred not to remove any of the unhybridised nucleic acid probes from the reaction mixture, i.e. all unhybridised nucleic acid probes remain in the reaction mixture during the incubation step, the connecting step and the amplifying step. It is however possible to remove a portion of the unhybridised nucleic acid probes from the mixture if desired. The skilled person is aware of suitable methods for such partial removal. By not removing any of the unhybridised nucleic acid probes from the reaction mixture, the method according to the invention, provides the possibility for an essential one-tube assay using more than 2 probes simultaneously and less than 10.000 copies of each target nucleic acid for each assay.

It is very attractive for the method of the present invention to be carried out as a "one tube" assay; i.e. the incubating step 1, the connecting step 2, the incubating step 3 and the amplification step 4 are carried out in the same reaction vessel in a sequential order, the reaction mixture not being removed from the said vessel during the said steps. Preferably at least the connecting step 2 and the incubating step 3 are performed simultaneously or sequentially.

The incubating step 1, the contacting step 2 and the incubation step 3 are usually carried out in a relatively small volume typically of 6-25 µl, although larger volumes, as well as increase of volume of the reaction mixture in subsequent reaction steps are tolerated. The amplification step 4 is usually performed in a volume of 20-30 µl.

Preferably, the reaction mixture comprises, at least during the step 2, connecting (ligation) activity, by which the first, second and optionally the third nucleic acid probes are, once hybridised to the target nucleic acid and arranged adjacently to one another, connected to one another.

In the step 3, the double-stranded connected probe assemblies - target sequence are subjected to digestion by the methylation sensitive restriction endonuclease. As it is important that the cleaved nucleic acids are not religated to one another, any ligation activity present, at least during the said step 3, is incapable of ligating double stranded nucleic acids. This can be done for example by inactivating the ligase activity from the step 2 before adding the methylation sensitive restriction endonulease, or, and preferably, a ligase activity is used, capable of ligating single-stranded nucleic acids to one another, such as the probes, hybridized to the target nucleic acid, while being incapable of ligating double-stranded nucleic acids. By using such a ligase activity, known in the art, for example NAD dependent ligases, the steps 2 and 3 can be performed simultaneously.

In another embodiment of the present invention said connection (ligation) of step 2 is performed with a thermostable nucleic acid ligase active at temperatures of typically 50°C, 60°C or higher, but capable of being rapidly inactivated above approximately 95°C. Once nucleic acid probes are connected it is preferred that essentially no connecting activity is present during amplification since this is not required and can only introduce ambiguity in the method. Since amplification steps usually require repeated denaturation of template nucleic acid at temperatures above 95°C it is preferred to remove the connecting activity through said heat incubation. In order to prevent hybridisation of probes to sequences only partially complementary it is preferred to perform the ligation reaction at temperatures of at least 45°C. It is however important, if the connecting step 2 is performed together with the incubation step 3, that the reaction temperature allows the required digestion to take place. The present invention therefore in one aspect provides a method wherein ligation of nucleic acid probes annealed to a target nucleic acid is performed by a thermostable nucleic acid ligation enzyme, i.e. with an activity optimum higher than at least 45°C, under suitable conditions, wherein at least 95% of the ligation activity of the said ligation enzyme is inactivated by incubating said sample for approximately 10 minutes at a temperature of approximately 95°C.

According to the present invention, the target nucleic acids present in the sample are not directly amplified, but only connected probe assemblies which have the complementary sequence of the target nucleic acid sequences. Target nucleic acid sequences originally found in the sample being analysed are not directly amplified because such target sequences do not contain amplification primer-specific tags. However, the nucleic acid sequence of the amplicon between the primers used for the amplification is identical to the target nucleic acid sequence.

The first and the second nucleic acid probes and the capture probes of the present invention, as well amplification primers, can be chemically synthesised. The suitable methods for chemical synthesis of oligonucleotides are known to those skilled in the art. Usually oligonucleotides are chemically synthesized using nucleotides, called phosphoramidites, normal nucleotides which have protection groups: preventing amine, hydroxyl groups and phosphate groups interacting incorrectly. One phophoramidite is added at a time, the product's 5' phosphate is deprotected and a new base is added and so on (backwards), at the end, all the protection groups are removed. Products are often isolated by HPLC to obtain the desired oligonucleotides in high purity. Chemically produced oligonucleotides are cheap, essentially pure and are available from many suppliers. Thus knowing the target nucleic acid sequence, it is easy for the skilled person in the art to prepare the MLM probes or capture probes of the invention.

The length of the complementary region with the target nucleic acid in the probe is preferably long enough to allow annealing at elevated temperatures. Typically the length of the complementary region is at least 10 nucleotides, preferably at least 18 nucleotides. The probes also contain a tag which can be of any size; however, typically a tag comprises a nucleic acid with a length of at least 15 nucleotides. A probe comprising a tag therefore typically comprises a length of 33 or more nucleotides. Amplicons of connected first and second nucleic acid probes typically have a length of at least 66 nucleotides. Said amplicons have substantially the same size (length). As herein used, the term "substantially the same size" refers to size (length) measured in number of nucleotides, wherein said size (length) of different oligonucleotides is "substantially the same" when it differs only in small number of nucleotides (for example only approximately 10 nucleotides). This difference is not sufficient to discriminate different oligonucleotides on the basis of size (length).

A problem, particularly encountered in multiplex amplifications, is the discrimination of the different amplicons that can result from the amplification step 4. Discrimination can be achieved in a number of different ways. According to the method of the present invention, amplicons of step 4 are discriminated between on the basis of the respective sequences present in the amplicon; for example through hybridising amplicons to specific probes. In the preferred embodiment of the present invention said specific probes are capture probes which are oligonucleotides having a sequence complementary to at least about half of the sequences of both the first and the second nucleic acid probes of each target nucleic acid sequence. The hybridization between amplicons and capture probes is based on the property of complementary nucleic acid sequences to specifically pair with each other by forming hydrogen bonds between complementary nucleotide base pairs. A high number of complementary base pairs in a nucleotide sequence means tighter non-covalent bonding between the two strands. After washing off of non-specific bonding sequences, only strongly paired strands will remain hybridized. Usually fluorescently labeled amplicons that bind to a capture probe sequence generate a signal that depends on the strength of the hybridization determined by the number of paired bases, the hybridization conditions (such as temperature), and washing after hybridization. Total strength of the signal, from a spot (feature), depends upon the amount of the amplicons binding to the capture probes present on that spot.

The capture probes are synthesized prior to immobilization and then immobilized (attached) to a membrane or to a solid surface by a covalent bond to a chemical matrix (for example via epoxy-silane, amino-silane, lysine, polyacrylamide or others) according to the methods known to those skilled in the art, for example Zhang, Y., Coyne, M., Will, S., Levenson, C., Kawasaki, E. 1991. Single base mutational analysis of cancer and genetic diseases using membrane bound modified oligonucleotides. Nuc. Acids. Res. 19: 3929-3933. The immobilizing of the capture probes to a membrane or to a solid support generates macroarray or microarray. The membrane is a membrane in a multichannel support, which can be for example, but not limited to, a nylon membrane. After stripping, these membranes can be reused 10-20 times without substantial loss of sensitivity. The solid support is, for example glass, plastic or a silicon chip, in which case they are commonly known as *gene chip* or colloquially *Affy chip* when an Affymetrix chip is used. Alternatively the captures probes as unmodified oligonucleotides can be spotted onto aminosilane-coated glass slides or the capture probes as amino-modified oligonucleotides can be spotted on silyated glass slides. The capture probes can be also spotted on any other suitable slide, solid surface or membrane.

Other microarray platforms for immobilizing (attaching) capture probes can be used, such as Illumina, which use microscopic beads, instead of the large solid support.

In several examples, the Applicants have obtained labelled amplification products by using a fluorescent amplification primer and detected the amplicons on the macroarray membrane with one colour fluorescent detection system. Some automatic fluorescence scanners rely on the use of four differently fluorescently labelled primers each having a unique colour signature, enabling the analysis of more than one sample in a single lane and the use of internal size standards. It is however also possible to use PCR primers which are radioactively labelled, or that are labelled with other compounds that can be detected with the use of the appropriate calorimetric or chemiluminescent substrates. In a clinical setting and for general use in many clinical testing laboratories, it is preferable that methods not requiring the use of radiolabeled nucleotides be used.

The fluorescent tags are described for instance by Lee et al (Nucleic Acid Research 21: 3761-3766 1993). Detection of fluorescence during the thermal cycling process can be performed for instance with the use of the ABI Prism 7700 sequence detection System of the PE Biosystems Corp. Other real time detection methods that do not rely on the destruction of sequence tag bound oligonucleotides by the 5' nuclease activity of a polymerase but on the increased fluorescence of some fluorogenic probes (molecular beacons) upon binding to the sequence tag can also be used in the present invention as well as detection probes consisting of two entities, each being complementary to sequences present on one or more amplification-products and each containing a fluorescent moiety wherein fluorescent resonance energy transfer (FRET) occurs upon binding of both entities to the target amplification product.

Fluorescent tags can be also fluorescent dyes commonly used for cDNA labeling include Cy3, which has a fluorescence emission wavelength of 570 nm (corresponding to the green part of the light spectrum), and Cy5 with a fluorescence emission wavelength of 670 nm (corresponding to the red part of the light spectrum).

Alternatively, mass spectrometry can be used to detect and identify the amplification products of step 4.

The level of methylation can be determined by calculating the ratio of the relative peak area of each target probe from the digested sample and from the undigested sample.

In another embodiment of the present invention, the plurality of different nucleic acids, present in a sample, are obtained from the same subject by denaturation and fragmentation of the genomic DNA from said subject. This provides a DNA print of one subject, which can be available on a support, such as a chip, to be read by a machine, for example a fluorescence scanner, in order to obtain the information for said subject. In this embodiment of the present invention, the capture probes, as described in the present invention, are immobilized on a solid support, such as glass, plastic or a silicon chip, with methods known in the art. Said capture probes are then hybridized with the amplicons tagged for example, but not limited to, a fluorescent tag (for example FAM, Cy3 or Cy5), which allows the reading with a fluorescence scanner.

Due to its simplicity, the Methylation Ligation-dependent Macroarray (MLM) method described herein could serve as a powerful screening tool in tumor classification where often only limited amounts of DNA are available from tissue slices that have been characterized by histological examination. MLM can be used for the analysis of both methylation as well as copy number changes in DNA derived from blood samples of patients with various disorders such as, but not limited to, cancer, abnormal embryonic development, polypus.

The Methylation Ligation-dependent Macroarray (MLM) can be also used in clinical trials to identify human profiles for appropriate drug delivery, for metastases prognosis, to make patients diagnosis and to distinguish pathologies with or without evolution.

A further application of the current invention is the detection of pathogens in a sample. There are many different pathogens that can contaminate food samples or be present in clinical samples. Determination of even minor quantities of a pathogen can be accomplished using nucleic acid amplification methods such as PCR, RT-PCR and 3SR. However, for these purposes, considering the wide variety of potential pathogens, a large number of different primer sets need to be used and their performance optimised. Although possible, this is a lengthy process. In addition, very often not all primer sets can be added in one reaction mix thus necessitating different reactions for full coverage of the potential pathogens. With the present invention it is possible to scan the presence or absence of a large number of different pathogens in one sample. This can be accomplished by analysing methylated DNA in a sample.

Several aspects contribute to the benefit of MLM method: (i) a large number of genes can be studied using a minimum amount of DNA, typically only 40 ng sample DNA; (ii) due to its simple procedure, large numbers of samples can be analyzed simultaneously, for example 50 samples; (iii) MLM is quantitative and can discriminate between methylation of one, both or none of the alleles; (iv) the optional simultaneous ligation and digestion reaction enables MLM to be used on paraffin-embedded tissue samples, because DNA degradation and partial DNA denaturation during embedding of the tissues or longtime storage appear not to influence the results; (v) the MLM probes are small, easy to synthesize and provide homogenous PCR amplification; (vi) the use of multichannel support of this invention provides a quick analysis of results and allows the re-use of this support.

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes which come within the meaning and range of equivalency are intended to be embraced therein.

The foregoing description will be more fully understood with reference to the following Examples. Such Examples, are, however, exemplary of methods of practising the present invention and are not intended to limit the scope of the invention.

### EXAMPLES

One example for carrying out the method of the present invention is described herein:

### Genomic DNA sonication

Genomic DNA is sonicated in an ultrasound bath during 30 minutes. A sample is analysed on a 2% agarose gel in order to check the DNA fragments size: it should be of about 300 - 500 nucleotides.

### 1st step: Incubating (annealing)

Samples are prepared in 0.2 ml tubes, a sample containing sonicated DNA (40 ng in 2 µl) is prepared on ice. Incubation (annealing) mix:

| | |
|---|---|
| H₂O | 5.0 µl |
| Mix of nucleic acid probes * | 1.5 µl |
| MLM buffer | 1.5 µl |
| Total | 8.0 µl |

| | |
|---|---|
| * 0.75 fmole of each nucleic acid probe Incubation in a thermocycler: Incubation: 98°C, 10 min, then Incubation: 60 °C, 16 h | |

### 2nd and 3rd step: Ligation (connections) / digestion (incubating)

Tubes are taken out of the thermocycler and put on ice:

In each tube, the following is add: 8.0 µl DNA solution, 3.0 µl ligase buffer A and 11.0 µl H₂O, the total being 21.0 µl. Each tube is divided into 2 x 10 µl.
10.0 µl of digestion mix are added in one tube (positive tube), whereas 10 µl of mix is added on the second tube (negative tube) as follows:

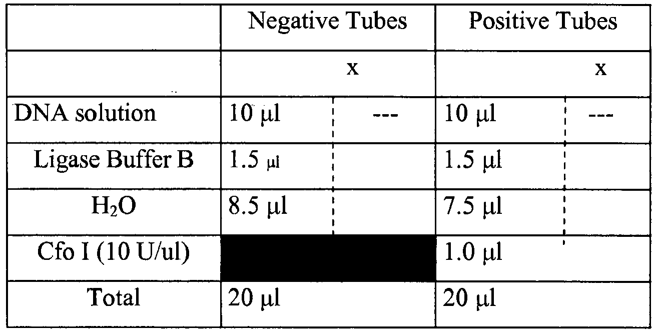

Tubes are incubated in the thermocycler: Incubation: 37°C, 1h, then Incubation: 60°C, 5 min. Samples are put on ice and 5.0 µl of ligation mix are added in all tubes.

### Ligation mix:

| | |
|---|---|
| DNA solution | 20 µl |
| H₂O | 3.625 µl |
| Ligase Buffer A | 0.75 µl |
| Ligase Buffer B | 0.375 µl |
| MLPA ligase | 0.25 µl |
| Total | 25 µl |

Tubes are incubated in the thermocycler: Incubation: 60°C, 15 min, then Incubation: 98°C, 5 min and then keep at 15°C (unlimited time).
Ligation mix is used immediately or frozen at -20°C.

### 4^{th} step: amplification and labelling

| | 1 tube contains |
|---|---|
| H₂O | 9,3µl |
| PCR buffer 10x-MgCl₂ | 2,0 µl |
| MLM-Fw-**biotin** (10 picomoles./µl) | 1.0 µl |
| MLM-Rev (10 picomoles/µl) | 1.0 µl |
| 50mM MgCl₂ | 0.6 µl |
| 5mM dNTP | 1.0 µl |
| Taq. pol. | 0.1 µl |
| Volume total | 15.0 µl |
| DNA solution obtained in step 3 | 5.0 µl |
| Final volume | 20.0 µl |

### Programme :

| | |
|---|---|
| | 95°C, 5 min. |
| 35 Cycles : | 94°C, 30sec. / 60 °C, 30 sec. / 72°C, 60sec. |
| | 72°C, 20 min. |
| | 15°C, forever |

*PCR product is verified on 2% agarose gel. If migration is correct , proceed to macroarray hybridization*

### 5^{th} step: Immobilizing capture probes (membrane preparation)

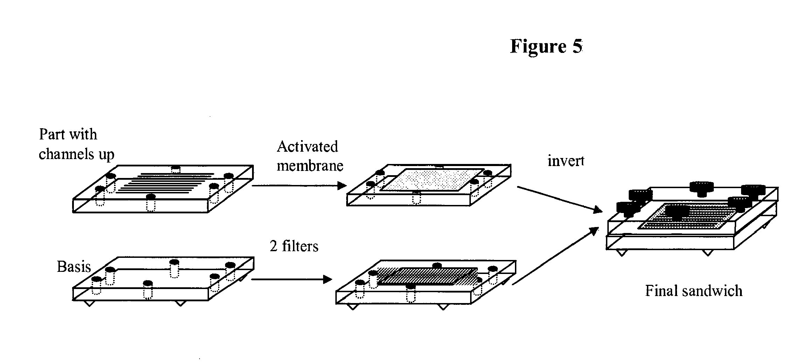

- In a 0.5 ml tube, the following is prepared: Probe 100 µM (5 µl) and 0.5M NaHCO₃ pH8.4 (195 µl) - the total volume being 200 µl *(No time limitation).* With gloves, a square 14cm x 14 cm is cut in Nylon membrane. Membrane is activated by immerging it in **16% EDAC** (prepared EX TEMPO in a Falcon 50 ml tube with nanopure water, 20 ml minimum to be prepared), in a sealed pocket (no bubbles), with agitation, 10 min, RT. Membrane is rinsed during 2 min. with nanopure water. Membrane is placed on 2 gaskets in miniblotter (assemble and soak liquid in channels - see Figure 5). Probes are loaded, without loading 1^{st} channel. If possible last channel is free as well (to be loaded with green ink). 17 µl Indian ink is loaded in 1^{st} channel. Soak at the other extremity to load all channel. When all probes are loaded, wait 1 min., RT. Probe is soaked using the same order that the one used for loading. Membrane is transferred in a glass recipient containing 250 ml of 10mM NaOH. Incubate 10 min. with agitation, RT, for inactivation. Membrane is washed twice in 250 ml 2x SSPE / 0.1 % SDS, 5 min., at 60°C with agitation and then washed in 100 ml 20mM EDTA pH8.0, RT, 15 min. with agitation. Membrane is stored at 4°C (fridge), in fresh 20mM EDTA pH8.0.

### MINIBLOTTER WASHES

Miniblotter is left in 0.1N NaOH / 1% SDS for a couple of hours. Channels are softly cleaned by brushing them with a teeth brush (channel direction),. washed twice with tap water, then washed twice with deionised water and finally washed with nanopure water. "Sandwich" is made by using an old gasket. Channels are filled up twice with 3% H₂O₂, at RT, 1h., then washed during 15-30 minutes with deionised water, then with nanopure water and finally dried with air.

### SOLUTIONS

### 0.5M NaHCO3 pH8.4

| | |
|---|---|
| NaHCO₃ (n°1.06329.0500, Merck) | 4.2 g |
| H₂O | |
| Total | 100 ml |

Adjust pH to 8.4 with Na OH 1M (about 1 ml)

### 16% EDAC EX TEMPO

| | |
|---|---|
| EDAC (n° 341006, Calbiochem) | 3.2 g |
| H₂O | |
| Total | 20 ml |

### Inks (stock at reception)

Black ink, Rotring R 591 217, 250 ml, waterproof
Tissue marking dye, Cancer diagnostics, INC #0728-3-Green, 237 ml

### NaOH 10mM

| | |
|---|---|
| NaOH 5M | 2 ml |
| H₂O | 998 ml |
| Total | 1000 ml |

### SSPE 20x

| | | |
|---|---|---|
| NaCl (Sigma, n° S3014) | 175.3 g | *(3M final)* |
| NaH₂PO₄, 2H₂O(Merck n° 1.06329) | 31.2 g | *(or 27.6 g using NaH₂PO₄, H₂O) (0.2M final)* |
| EDTA (Calbiochem, n° 324503) | 7.4 g | *(0.02M final)* |
| H₂O | | |
| Total | 1000 ml | |

Adjust pH to 7.4 with NaOH 5M
Autoclave, and keep at RT.

### SSPE 2x / SDS 0.1% (for one membrane)

| | |
|---|---|
| SSPE 20x | 50 ml |
| SDS 10% | 5 ml |
| H₂O | 445 ml |
| Total | 500 ml |

### 20 mM EDTA pH8.0

| | |
|---|---|
| EDTA 0.5M pH8.0 (Sigma, n° E-7889) | 20 ml |
| H₂O | 480 ml |
| Total | 500 ml |

### 0.1N NaOH / 1% SDS

| | |
|---|---|
| NaOH | 8 g |
| SDS | 20 g |
| H₂O | |
| Total | 2000 ml |

### 3% H₂O₂

| | |
|---|---|
| H₂O₂ 30% | 1.5 ml |
| H₂O | |
| Total | 15.0 ml |

### 6^{th} step: Macroarray hybridization

All buffers should be pre-heated at 60°C.

### 1. Hybridization (at 60°C)

Samples in 0.5 ml tube are prepared as follows: :

| | |
|---|---|
| PCR product | 5 µl + 5 µl 1 dye bleu 1/2 |
| Nanopure H₂O | 95 µl |
| Total | 105 µl |

with short spin, denaturation 94°C, 5 min. in heatting block. Tubes are immediately put at 4°C. Membrane is taken out from its conserving liquid and then placed on a new gasket in miniblotter, inked lines perpendicular to channels. Miniblotter is assembled and remaining liquid is soaked in channels.
All channels are filled with pre-heated (60°C) 2x SSPE / 0.1%SDS (*1 channel = 150* µl maximum, without bubbles). 110 µl 8x SSPE / 0.4% SDS, pre-heated at 60°C is added on samples. Channels are soaked, and the 1^{st} channel is loaded using pipette 20-200 µl. The following channels are soaked, the 2^{nd}, channel is loaded, and etc... Liquid present in other channels will protect them from cross-contamination. When all PCR products are loaded, a piece of coloured rubber band is added at each extremity of channels to close them and to avoid evaporation. The the incubation is performed during 1h at 60°C in hybridization oven.

### 2. Washes (at 63°C, oven on table)

The liquid is soaked in channels. The membrane is taken, wrapped into a hybridization sheet and transferred in a small hybridization bottle. Then it is washed twice during 15 min. at 63°C with rotation ~30-50ml 2x SSPE / 0.5% SDS preheated at 63°C.

### 3. Pre-detection (at RT)

Anti-Biotin: 1x 30 min, 30 ml (Streptavidine-AP 3 µl / 2xSSPE / 0.5% SDS) at RT (Room Temperature)

### 4. Detection : chemiluminescence

Membrane is put into a transparent small plastic tank. Then it is washed two times during 10 min. with 50 ml (2x SSPE/0.5% SDS) at RT. The membrane is then put in a new plastic tank (to remove SDS) and washed again two times during 5 min. with 50 ml (2x SSPE) at RT and two times during 2 min. with 20 ml Buffer IIIA at RT. The membrane is then put on a plastic bag.
Staining: the membrane is covered with CDP star Buffer "ready-to-use", and incubated during 5 min. light-protected. The solution is removed and the plastic bag is sealed.
The membrane is exposed on a Amersham Biosciences, Hyperfilm ECL, #RPN1677K film.

### 5. Stripping

Membrane is washed twice with 100 ml hot 1% SDS during 40 min. in a glass tank. Then it is rinsed in 100 ml 20 mM EDTA pH 8.0 during 15 min. at RT in small plastic transparent tank. After rinsing, the membrane is put in a plastic pocket and exposed to a film to check absence of signal. Finally, the membrane is stored in new 20 mM EDTA pH8.0 at 4°C.

### 6. Miniblotter washes

The miniblotter is left in 0.1N NaOH / 1% SDS for a couple of hours. Then it is softy cleaned by brushing is with a teeth brush (channel direction). After brushing, the miniblotter is washed twice with tap water, then twice with deionised water and finally with nanopure water. Sandwich is made using an old gasket. The channels are filled up two times with 3% H₂O₂, at RT, 1h. *(crucial to eliminate background)* and then washed during 15-30 min. with deionised water and with nanopure water. Finally channels are dried with air.

### SOLUTIONS

### Dye MLM

| | |
|---|---|
| Ficoll 20% | 450 µl |
| Xylene Cyanol 1% | 60 µl |
| Bromophenol Blue 1% | 60 µl |
| H₂O | 430 µl |
| Total | 1000 µl |

### 8x SSPE / 0.4% SDS

| | |
|---|---|
| SSPE 20x | 4.0 ml |
| SDS 10% | 0.4 ml |
| H₂O | 5.6 ml |
| Total | 10 ml |

### 2x SSPE / 0.1%SDS

| | |
|---|---|
| SSPE 20x | 50 ml |
| SDS 10% | 5 ml |
| H₂O | 445 ml |
| Total | 500 ml |

### 2x SSPE / 0.5% SDS

| | |
|---|---|
| SSPE 20x | 200 ml |
| SDS 10% | 100 ml |
| H₂O | 1700 ml |
| Total | 2000 ml |

### Streptavidine-AP 1 ul / 2x SSPE / 0.5% SDS

### 2x SSPE / 0.5% SDS 20 ml

| | |
|---|---|
| Streptavidine-AP | 2 µl |
| Total | 20 ml |

### 2x SSPE

| | |
|---|---|
| SSPE 20x | 100 ml |
| H₂O | 900 ml |
| Total | 1000 ml |

### 0.1N NaOH / 1% SDS

| | |
|---|---|
| NaOH | 8 g |
| SDS | 20 g |
| H₂O | |
| Total | 2000 ml |

### 3% H₂O₂

| | |
|---|---|
| H₂O₂ 30% | 1.5 ml |
| H₂O | |
| Total | 1̅5̅.̅0̅ m̅l̅ |

Here below are disclosed examples of nucleic acid probes and primers used for the detection of several genes with the MLM method of the present invention:
"MLM-XXX-A" corresponds to the first nucleic acid probes
"MLM-XXX-Bp" corresponds to the second nucleic acid probes, being phosphorylated at the 5' extremity.
"MLM-XXX-P" corresponds to the capture probes having an amino function at 5' extremity.
"XXX" corresponds to a gene (target nucleic acid sequence) on which the first nucleic acid probe and the second nucleic acid probe are hybridized.

In all nucleic acid sequences disclosed here-below, the caps letters indicate tags added either at the 5' extremity ("MLM-...-A"), or at the 3' extremity ("MLM-...-Bp"). These tags are specific for the nucleic acid sequence of the amplification primers MLM-Fw and MLM-Rev.

| SEQ ID NO | | |
|---|---|---|
| 1 | *MLM-ACTIN-A* | *GGGTTCCCTAAGGGTTGGAccctgaggcactcttccagc* |
| 2 | *MLM-ACTIN-Bp* | *cttccttcctgggtgagtggagTCTAGATTGGATCTTGCTGGCAC* |
| 3 | *MLM-ACTIN-P* | *ggaaggaaggctggaagagtgc* |
| 4 | *MLM-CALCA-A* | *GGGTTCCCTAAGGGTTGGAcacagcggcgggaataagagca* |
| 5 | *MLM-CALCA-Bp* | *gtcgctgGCGCtgggaggTCTAGATTGGATCTTGCTGGCAC* |
| 6 | *MLM-CALCA-P* | *cgccagcgactgctcttattcc* |
| 7 | *MLM-CDKN2A-A* | *GGGTTCCCTAAGGGTTGGAgggggaGCGCggctggg* |
| 8 | *MLM-CDKN2A-Bp* | *agcagggaggccggagggTCTAGATTGGATCTTGCTGGCAC* |
| 9 | *MLM-CDKN2A -P* | *gcctccctgctcccagccg* |
| 10 | *MLM-ESR1-A* | *GGGTTCCCTAAGGGTTGGAgggacatGCGCtgcgtcg* |
| 11 | *MLM-ESR1-Bp* | *cctctaacctcgggctgtgcTCTAGATTGGATCTTGCTGGCAC* |
| 12 | *MLM-ESR1-P* | *gaggttagaggcgacgcagcg* |
| 13 | MLM-MGMT-A | GGTTCCCTAAGGGTTGGAgtggtcctgcagGCGCcc |
| 14 | MLM-MGMT-Bp | tcacttcgccgtcgggtgtgTCTAGATTGGATCTTGCTGGCAC |
| 15 | MLM-MGMT-P | cgaagtgagggcgcctgcag |
| 16 | *MLM-MLH1-A* | *GGGTTCCCTAAGGGTTGGAccgctcgtagtattcgtgctcag* |
| 17 | *MLM-MLH1-Bp* | *cctcgtagtgGCGCctgacgTCTAGATTGGATCTTGCTGGCAC* |
| 18 | *MLM-MLH1-P* | *ccactacgaggctgagcacgaa* |
| 19 | *MLM-p73-A* | *GGGTTCCCTAAGGGTTGGAcctactccccgcgGCGCct* |
| 20 | *MLM-P73-Bp* | *cccctccccGCGCccatatTCTAGATTGGATCTTGCTGGCAC* |
| 21 | *MLM-p73-P* | *ggggaggggaggcgccgcg* |
| 22 | MLM-RARb-A | GGGTTCCCTAAGGGTTGGActtgtGCGCtcgctgcctg |
| 23 | MLM-RARb-Bp | cctctctggctgtctgcttttgTCTAGATTGGATCTTGCTGGCAC |
| 24 | MLM-RARb-P | ccagagaggcaggcagcgag |
| 25 | *MLM-RASSF1A-A* | *GGGTTCCCTAAGGGTTGGAggtttccattGCGCggctctc* |
| 26 | *MLM-RASSF1A-Bp* | *ctcagctccttcccgccgcTCTAGATTGGATCTTGCTGGCAC* |
| 27 | *MLM-RASSF1A-P* | *ggaaggagctgaggagagccg* |
| 28 | *MLM-TIMP3-A* | *GGGTTCCCTAAGGGTTGGAgagcgggcagcaggcagg* |
| 29 | *MLM-TIMP3-Bp* | *cggcggGCGCtcagacggTCTAGATTGGATCTTGCTGGCAC* |
| 30 | *MLM-TIMP3-P* | *cgcccgccgcctgcctgc* |
| 31 | *MLM-Wnt2-A* | *GGGTTCCCTAAGGGTTGGAtcccggagctgaGCGCttc* |
| 32 | *MLM-Wnt2-Bp* | *tgctctgggcacgcatggcTCTAGATTGGATCTTGCTGGCAC* |
| 33 | *MLM-Wnt2-P* | *gcgtgcccagagcagaagcgc* |

### PCR amplification primers:

| | | |
|---|---|---|
| MLM-Fw | GGGTTCCCTAAGGGTTGGA | (SEQ ID NO: 34) |
| MLM-Rev | GTGCCAGCAAGATCCAATCTAGA | (SEQ ID NO: 35) |

## Claims

1. Method for detecting in a sample comprising at least one nucleic acid, the presence of at least one methylated site on a specific location in a specific single stranded target nucleic acid sequence comprising a first and a second segment, said segments located adjacent to one another, and said method comprising, in a reaction mixture, the steps of:
1) incubating said sample, comprising at least one nucleic acid, with a plurality of different probe sets, under conditions allowing hybridization between complementary target nucleic acid sequences in said nucleic acid with said probe sets, each probe set comprising
a first nucleic acid probe having
a first target specific region complementary to the first segment of said target nucleic acid sequence and
a first non-complementary region, 3' from the first region, being non-complementary to said target nucleic acid sequence, and comprising a tag sequence,
a second nucleic acid probe having
a second target specific region complementary to the second segment of said target nucleic acid sequence, phosphorylated at the 5' extremity, and
a second non-complementary region, 5' from the second region, being non-complementary to said target nucleic acid sequence, and comprising a tag sequence,
and obtaining said first and said second nucleic acid probe of each probe set being hybridized to the complementary first and second segment of the same target nucleic acid sequence, respectively said hybridized first and second nucleic acid probes of each probe set being located adjacent to one another,
2) connecting to one another the first and the second nucleic acid probes hybridized to target nucleic acid sequences, which provides a plurality of different connected probe assemblies of substantially the same size, and forming a double-stranded connected probe assemblies - target sequence, wherein the sequence of at least one of the nucleic acid probes is chosen such that, in the connected probe assembly, at least one double-stranded recognition site for a methylation sensitive restriction enzyme is present,
3) incubating said connected probe assemblies - target sequence with at least one methylation sensitive restriction enzyme, allowing said methylation sensitive restriction enzyme to cleave said double-stranded connected probe assemblies - target sequence at unmethylated recognition sites, leaving methylated recognition sites intact,
4) amplifying the connected probe assemblies, wherein amplification is initiated by binding of a first amplification primer specific for a tag sequence of said first nucleic acid probe, followed by elongation, whereby producing amplicons of substantially the same size
5) immobilizing capture probes, having a sequence complementary to at least about half of the sequences of both the first and the second nucleic acid probes, to a support,
6) contacting simultaneously all amplicons of step 4 with said capture probes under conditions effective to hybridize said amplicons to said capture probes.
7) detecting said amplicons, wherein said detecting indicates the presence in the sample of one or more methylated sites on specific location in specific single stranded target nucleic acid sequence.

2. The method of claim 1, wherein said sample comprises a plurality of different nucleic acids.

3. The method of claims 1 - 2, wherein said nucleic acids have homogenized sizes and are obtained by sonication of the genomic DNA in an ultrasounds bath.

4. The method of claims 1 - 3, wherein at least steps 2 and 3 are performed simultaneously or sequentially.

5. The method of claims 1 - 4, wherein the amplification step 4 comprises binding of a second amplification primer, specific to a tag sequence of said second nucleic acid probe, to the elongation product of the first primer.

6. The method of claims 1 - 5, wherein steps 1 to 4 are carried out in the same reaction vessel in a sequential order.

7. The method claims 1 - 6, wherein said first nucleic acid amplification primer and/or said second nucleic acid amplification primer is tagged with a labelled tag.

8. The method of claim 7, wherein said labelled tag is selected in the group comprising fluorescent tag, non-fluorescent tag, radioactive tag, luminescent tag and phosphorescent tag.

9. The method of claims 1 - 8, wherein said sample comprises a plurality of different nucleic acids obtained from the same subject.

10. The method of claims 1 - 9, wherein the support of step 5 is a membrane in a multichannel support or a solid support.

## Patentansprüche

1. Verfahren zum Nachweis in einer Probe, umfassend mindestens eine Nukleinsäure, die Anwesenheit von mindestens einem methylierte Stelle an einer bestimmten Position in einem spezifischen einzelsträngigen Ziel-Nukleinsäuresequenz, umfassend ein erstes und ein zweites Segment, wobei die Segmente benachbart zueinander, und wobei das Verfahren umfasst, in einer Reaktionsmischung, die folgenden Schritte:
1) Inkubieren der Probe, umfassend mindestens eine Nukleinsäure, die mit einer Vielzahl von verschiedenen Sondensätzen, unter Bedingungen, die eine Hybridisierung zwischen komplementären Ziel-Nukleinsäuresequenzen in der Nukleinsäure mit dem Sondensätze, wobei jeder Sondensatz, umfassend
eine erste Nukleinsäure-Sonde mit
eine erste zielspezifische Region komplementär zu dem ersten Segment der Zielnukleinsäuresequenz und
einer ersten nicht-komplementären Region, 3' von der ersten Region, nichtkomplementär zu der Ziel-Nukleinsäuresequenz, und mit einer Markierungssequenz,
eine zweite Nukleinsäure-Sonde mit
einer zweiten zielspezifische Region komplementär zu dem zweiten Segment der Zielnukleinsäuresequenz, 5' Ende phosphoryliert, und
eine zweite nicht-komplementäre Region, 5' von der zweiten Region, nichtkomplementär zu der Ziel-Nukleinsäuresequenz, und mit einer Markierungssequenz,
und zum Erhalten der ersten und der zweiten Nukleinsäure-Sonde jedes Sondensatzes mit dem komplementären ersten und zweiten Segment des gleichen Ziel-Nukleinsäuresequenz hybridisiert, die jeweils der hybridisierten ersten und zweiten Nukleinsäure-Sonden eines jeden Sondensatz benachbart zueinander angeordnet sind,
2) verbinden zueinander der ersten und der zweiten Nukleinsäure-Sonden hybridisiert zum Ziel-Nukleinsäuresequenzen, die bildet eine Vielzahl von verschiedenen verbundenen Sondenmontagen im wesentlichen die gleiche Größe und bildet ein doppelsträngiges verbundenen Sondenmontagen - Zielsequenz, wobei die Sequenz von mindestens einer der Nukleinsäuresonden wird so gewählt, dass im verbundenen Sondenmontage, die mindestens eine Doppelstrang-Erkennungsstelle für eine methylierungssensitiven Restriktionsenzym vorhanden ist,
3) Inkubieren des verbundenen Sondenmontagen - Zielsequenz mit mindestens einem methylierungssensitiven Restriktionsenzym, so dass das methylierungssensitiven Restriktionsenzym zur Spaltung der Doppelstrang verbundenen Sondenmontagen - Zielsequenz an nicht-methylierte Erkennungsstellen, so dass methylierte Erkennungsstellen intakt,
4) Verstärkung der verbundenen Sondenanordnungen, wobei die Amplifikation initiiert durch Binden eines ersten Amplifikationsprimers spezifisch für eine Markierungssequenz des ersten Nukleinsäure-Sonde, gefolgt von einer Dehnung, wodurch die Herstellung Amplikons im wesentlichen die gleiche Größe,
5) Immobilisierung von Einfang-Sonden, mit einer Sequenz komplementär zu mindestens etwa der Hälfte der Sequenzen der beiden ersten und zweiten Nukleinsäure-Sonden, auf einem Träger,
6) gleichzeitiges Kontaktieren aller Amplikons aus Schritt 4 mit dem Einfang-Sonden unter Bedingungen, die Hybridisierung der Amplikons an die Einfang-Sonden.
7) Nachweis der besagten Amplikons, wobei die Erfassungs das Vorhandensein in der Probe eine oder mehrere methylierte Stellen auf bestimmte Position in spezifischen einzelsträngigen Ziel-Nukleinsäuresequenz.

2. Verfahren nach Anspruch 1, wobei die Probe eine Vielzahl von verschiedenen Nukleinsäuren umfasst.

3. Verfahren nach den Ansprüchen 1 - 2, wobei die Nukleinsäuren homogenisiert Größen und durch Beschallung der genomischen DNA in einem Ultraschall-Bad erhalten werden.

4. Verfahren nach den Ansprüchen 1 - 3, wobei mindestens die Schritte 2 und 3 werden gleichzeitig oder nacheinander durchgeführt.

5. Verfahren nach den Ansprüche 1 - 4. wobei die Amplifikationschritte 4 umfasst die Bindung eines zweiten Amplifikationsprimers, die spezifisch für eine Markierungssequenz der zweiten Nukleinsäure-Sonde, um das Verlängerungsprodukt des ersten Primers.

6. Verfahren nach den Ansprüchen 1 - 5, wobei die Schritte 1 bis 4 sind in dem gleichen Reaktionsgefäß in einer sequentiellen Reihenfolge durchgeführt.

7. Verfahren nach den Ansprüchen 1 - 6, wobei die erste Nukleinsäure-Amplifikationsprimer und / oder die zweite Nukleinsäure-Amplifikationsprimer mit einem markierten Markierung markiert ist.

8. Verfahren nach Anspruch 7, wobei die markierte Markierung ausgewählt ist aus der Gruppe bestehend aus die Fluoreszenzmarkierung, die nicht-Fluoreszenzmarkierung, der radioaktive Marker, die Lumineszenzmarkierung und die Phosphoreszenzmarkierung.

9. Verfahren nach den Ansprüchen 1-8, wobei die Probe eine Vielzahl von verschiedenen von der gleichen Person erhalten Nukleinsäuren umfasst.

10. Verfahren nach den Ansprüchen 1-9, wobei der Träger aus Schritt 5 ist eine Membran, die in einer Mehrkanal-Träger oder ein fester Träger ist.

## Revendications

1. Procédé pour détecter dans un échantillon comprenant au moins un acide nucléique, la présence d'au moins un site méthylé sur un emplacement spécifique dans une séquence spécifique d'un seul brin d'acide nucléique cible comprenant un premier et un second segment, lesdits segments adjacents les uns aux autres, et ledit procédé comprenant, dans un mélange réactionnel, les étapes de :
1) l'incubation dudit échantillon, comprenant au moins un acide nucléique, avec une pluralité de différents ensembles de sondes, dans des conditions permettant l'hybridation entre les séquences cibles complémentaires d'acides nucléiques dans ledit acide nucléique avec lesdits ensembles de sondes, chaque ensemble de sondes comprenant
une première sonde d'acide nucléique ayant
une première région spécifique à la cible et complémentaire au premier segment de ladite séquence d'acide nucléique cible et
une première région non complémentaire, 3' depuis la première région, étant non complémentaire à ladite séquence d'acide nucléique cible et comprenant une séquence de marquage,
une deuxième sonde d'acide nucléique ayant
une seconde région spécifique à la cible et complémentaire au deuxième segment de ladite séquence d'acide nucléique cible, phosphorylée au niveau de l'extrémité 5' et
une seconde région non complémentaire, 5' depuis la deuxième région, étant non complémentaire à ladite séquence d'acide nucléique cible et comprenant une séquence de marquage,
et l'obtention de ladite première et ladite deuxième sonde d'acide nucléique de chaque ensemble de sondes hybridée avec le premier et le deuxième segment complémentaire de la même séquence d'acide nucléique cible, respectivement lesdites première et seconde sondes hybridées d'acide nucléique de chaque ensemble de sondes étant adjacentes l'une à l'autre,
2) reliant entre elles les première et seconde sondes d'acide nucléique hybridées à des séquences cibles d'acides nucléiques, ce qui fournit une pluralité de différents assemblages de sondes connectées de sensiblement même taille, et la formation d'un double brin fait d'assemblages de sondes liées et de séquence cible, où la séquence d'au moins l'une des sondes d'acide nucléique est choisie de telle sorte que, dans l'assemblage de sonde connecté, au moins un site à double brin pour la reconnaissance par une enzyme de restriction sensible à la méthylation est présent,
3) l'incubation desdits assemblages de sondes connectées - séquence cible avec au moins une enzyme de restriction sensible à la méthylation, ce qui permet ladite enzyme de restriction sensible à la méthylation de cliver lesdits double brin fait d'assemblages de sondes connectées et séquence cible à des sites de reconnaissance non méthylés, en laissant des sites de reconnaissance méthylés intacts,
4) l'amplification des assemblages de sondes connectées, dans lequel l'amplification est initiée par la liaison d'une première amorce d'amplification spécifique à la séquence de marquage de ladite première sonde d'acide nucléique, suivie de l'élongation, de sorte que les amplicons de sensiblement même taille sont produits,
5) l'immobilisation des sondes de capture ayant une séquence complémentaire à au moins environ la moitié des séquences à la fois de la première et de la deuxième sondes d'acide nucléique, sur un support,
6) mettre en contact simultanément tous les amplicons de l'étape 4 avec lesdites sondes de capture, dans des conditions efficaces pour hybrider lesdits amplicons avec lesdites sondes de capture.
7) la détection desdits amplicons, où ladite détection indique la présence dans l'échantillon d'un ou plusieurs sites méthylés sur l'emplacement spécifique dans une séquence spécifique d'un seul brin d'acide nucléique cible.

2. Le procédé selon la revendication 1, où ledit échantillon comprend une pluralité d'acides nucléiques différents.

3. Le procédé selon les revendications 1-2, où lesdits acides nucléiques ont des tailles homogénéisés et sont obtenus par sonication de l'ADN génomique dans un bain d'ultrasons.

4. Le procédé selon les revendications 1 à 3, où au moins les étapes 2 et 3 sont effectuées simultanément ou séquentiellement.

5. Le procédé selon les revendications 1 à 4, où l'étape 4 d'amplification comprend liaison d'une seconde amorce d'amplification, spécifique à une séquence de marquage de ladite seconde sonde d'acide nucléique, au produit d'élongation de la première amorce.

6. Le procédé selon les revendications 1 à 5, où les étapes 1 à 4 sont réalisées dans le même récipient de réaction, dans un ordre séquentiel.

7. Le procédé revendique 1 - 6, où ladite amorce d'amplification du premier acide nucléique et / ou ladite amorce d'amplification du deuxième acide nucléique est marqué avec une étiquette de marquage.

8. Le procédé selon la revendication 7, où ladite étiquette de marquage est sélectionnée dans le groupe comprenant marqueur fluorescent, marqueur non fluorescent, marqueur radioactif, marqueur luminescent et marqueur phosphorescent.

9. Le procédé selon les revendications 1 à 8, où ledit échantillon comprend une pluralité de différents acides nucléiques obtenus du même sujet.

10. Le procédé selon les revendications 1 à 9, où le support de l'étape 5 est une membrane dans un support multi-canal ou un support solide.


### SEQUENCE LISTING

<110> CENTRE HOSPITALIER UNIVERSITAIRE VAUDOIS
<120> METHYLATION LIGATION-DEPENDENT MACROARRAY (MLM)
<130> 17317-PCT
<160> 35
<170> PatentIn version 3.3
<210> 1
   <211> 39
   <212> DNA
   <213> Artificial
<220>
   <223> synthetic sequence
<400> 1
   gggttcccta agggttggac cctgaggcac tcttccagc 39
<210> 2
   <211> 45
   <212> DNA
   <213> Artificial
<220>
   <223> synthetic sequence
<400> 2
   cttccttcct gggtgagtgg agtctagatt ggatcttgct ggcac 45
<210> 3
   <211> 22
   <212> DNA
   <213> Artificial
<220>
   <223> synthetic sequence
<400> 3
   ggaaggaagg ctggaagagt gc 22
<210> 4
   <211> 41
   <212> DNA
   <213> Artificial
<220>
   <223> synthetic sequence
<400> 4
   gggttcccta agggttggac acagcggcgg gaataagagc a 41
<210> 5
   <211> 41
   <212> DNA
   <213> Artificial
<220>
   <223> synthetic sequence
<400> 5
   gtcgctggcg ctgggaggtc tagattggat cttgctggca c 41
<210> 6
   <211> 22
   <212> DNA
   <213> Artificial
<220>
   <223> synthetic sequence
<400> 6
   cgccagcgac tgctcttatt cc 22
<210> 7
   <211> 36
   <212> DNA
   <213> Artificial
<220>
   <223> synthetic sequence
<400> 7
   gggttcccta agggttggag ggggagcgcg gctggg 36
<210> 8
   <211> 41
   <212> DNA
   <213> Artificial
<220>
   <223> synthetic sequence
<400> 8
   agcagggagg ccggagggtc tagattggat cttgctggca c 41
<210> 9
   <211> 19
   <212> DNA
   <213> Artificial
<220>
   <223> synthetic sequence
<400> 9
   gcctccctgc tcccagccg 19
<210> 10
   <211> 37
   <212> DNA
   <213> Artificial
<220>
   <223> synthetic sequence
<400> 10
   gggttcccta agggttggag ggacatgcgc tgcgtcg 37
<210> 11
   <211> 43
   <212> DNA
   <213> Artificial
<220>
   <223> synthetic sequence
<400> 11
   cctctaacct cgggctgtgc tctagattgg atcttgctgg cac 43
<210> 12
   <211> 21
   <212> DNA
   <213> Artificial
<220>
   <223> synthetic sequence
<400> 12
   gaggttagag gcgacgcagc g 21
<210> 13
   <211> 36
   <212> DNA
   <213> Artificial
<220>
   <223> synthetic sequence
<400> 13
   ggttccctaa gggttggagt ggtcctgcag gcgccc 36
<210> 14
   <211> 43
   <212> DNA
   <213> Artificial
<220>
   <223> synthetic sequence
<400> 14
   tcacttcgcc gtcgggtgtg tctagattgg atcttgctgg cac 43
<210> 15
   <211> 20
   <212> DNA
   <213> Artificial
<220>
   <223> synthetic sequence
<400> 15
   cgaagtgagg gcgcctgcag 20
<210> 16
   <211> 42
   <212> DNA
   <213> Artificial
<220>
   <223> synthetic sequence
<400> 16
   gggttcccta agggttggac cgctcgtagt attcgtgctc ag 42
<210> 17
   <211> 43
   <212> DNA
   <213> Artificial
<220>
   <223> synthetic sequence
<400> 17
   cctcgtagtg gcgcctgacg tctagattgg atcttgctgg cac 43
<210> 18
   <211> 22
   <212> DNA
   <213> Artificial
<220>
   <223> synthetic sequence
<400> 18
   ccactacgag gctgagcacg aa 22
<210> 19
   <211> 38
   <212> DNA
   <213> Artificial
<220>
   <223> synthetic sequence
<400> 19
   gggttcccta agggttggac ctactccccg cggcgcct 38
<210> 20
   <211> 42
   <212> DNA
   <213> Artificial
<220>
   <223> synthetic sequence
<400> 20
   cccctccccg cgcccatatt ctagattgga tcttgctggc ac 42
<210> 21
   <211> 19
   <212> DNA
   <213> Artificial
<220>
   <223> synthetic sequence
<400> 21
   ggggagggga ggcgccgcg 19
<210> 22
   <211> 38
   <212> DNA
   <213> Artificial
<220>
   <223> synthetic sequence
<400> 22
   gggttcccta agggttggac ttgtgcgctc gctgcctg 38
<210> 23
   <211> 45
   <212> DNA
   <213> Artificial
<220>
   <223> synthetic sequence
<400> 23
   cctctctggc tgtctgcttt tgtctagatt ggatcttgct ggcac 45
<210> 24
   <211> 20
   <212> DNA
   <213> Artificial
<220>
   <223> synthetic sequence
<400> 24
   ccagagaggc aggcagcgag 20
<210> 25
   <211> 40
   <212> DNA
   <213> Artificial
<220>
   <223> synthetic sequence
<400> 25
   gggttcccta agggttggag gtttccattg cgcggctctc 40
<210> 26
   <211> 42
   <212> DNA
   <213> Artificial
<220>
   <223> synthetic sequence
<400> 26
   ctcagctcct tcccgccgct ctagattgga tcttgctggc ac 42
<210> 27
   <211> 21
   <212> DNA
   <213> Artificial
<220>
   <223> synthetic sequence
<400> 27 21
   ggaaggagct gaggagagcc g 21
<210> 28
   <211> 37
   <212> DNA
   <213> Artificial
<220>
   <223> synthetic sequence
<400>- 28
   gggttcccta agggttggag agcgggcagc aggcagg 37
<210> 29
   <211> 41
   <212> DNA
   <213> Artificial
<220>
   z2235 synthetic sequence
<400> 29
   cggcgggcgc tcagacggtc tagattggat cttgctggca c 41
<210> 30
   <211> 18
   <212> DNA
   <213> Artificial
<220>
   <223> synthetic sequence
<400> 30
   cgcccgccgc ctgcctgc 18
<210> 31
   <211> 38
   <212> DNA
   <213> Artificial
<220>
   <223> synthetic sequence
<400> 31
   gggttcccta agggttggat cccggagctg agcgcttc 38
<210> 32
   <211> 42
   <212> DNA
   <213> Artificial
<220>
   <223> synthetic sequence
<400> 32
   tgctctgggc acgcatggct ctagattgga tcttgctggc ac 42
<210> 33
   <211> 21
   <212> DNA
   <213> Artificial
<220>
   <223> synthetic sequence
<400> 33
   gcgtgcccag agcagaagcg c 21
<210> 34
   <211> 19
   <212> DNA
   <213> Artificial
<220>
   <223> synthetic sequence
<400> 34
   gggttcccta agggttgga 19
<210> 35
   <211> 23
   <212> DNA
   <213> Artificial
<220>
   <223> synthetic sequence
<400> 35
   gtgccagcaa gatccaatct aga 23